# EUROPÄISCHE PATENTSCHRIFT

(11) **EP 2 799 020 B1**
(45) Veröffentlichungstag und Bekanntmachung des Hinweises auf die Patenterteilung: **22.01.2020**
(21) Anmeldenummer: 14001383.0
(22) Anmeldetag: 16.04.2014
(51) Int. Cl.: A61B 17/32, A61B 17/29

(54) **Chirurgisches Werkzeug und mikrochirurgisches Instrument**
Surgical tool and microsurgical instrument
Outil chirurgical et instrument de microchirurgie

(30) Priorität: 29.04.2013 DE 102013007315
(43) Veröffentlichungstag der Anmeldung: 05.11.2014
(60) Teilanmeldung: 19000550.4
(73) Patentinhaber: Karl Storz SE & Co. KG, 78532 Tuttlingen (DE)
(72) Erfinder: Frings, Hermann-Josef, 52072 Aachen (DE)
(74) Vertreter: Drobnik, Stefanie

(56) Entgegenhaltungen:
- EP-A1- 2 392 270
- DE-U1-202007 009 310
- GB-A- 2 161 707
- US-A- 4 848 338
- US-B1- 8 241 228

## Beschreibung

Die nachfolgende Erfindung bezieht sich auf ein chirurgisches Werkzeug und ein mikrochirurgisches Instrument.

In der minimalinvasiven Chirurgie werden chirurgische Werkzeuge und mikrochirurgische Instrumente verwendet, bei deren Nutzung der Operateur nur ein 2D-Bild des Operationsfeldes sieht, weshalb er besonders auf seinen Tastsinn angewiesen ist. Er kann das Gewebe im Operationsfeld jedoch nicht direkt ertasten, sondern nur indirekt und ist dabei auf Endoskope oder Laparoskope, die am Ende eines Schafts das chirurgische Werkzeug tragen, angewiesen. Dabei können als chirurgische Werkzeuge Greifer, Zangen oder Scheren mit beweglichen Branchen zum Einsatz kommen.

Um Gewebeschädigungen infolge zu großer Krafteinwirkung zu vermeiden, können diese Instrumente dem Operateur eine Rückmeldung über die eingesetzte Kraft liefern (Force Feedback). Solche Instrumente werden an sich rein mechanisch bedient, d. h., die Kraft, die der Operateur auf die Betätigungsvorrichtung ausübt, wird über Bowdenzüge oder Schubstangen durch den Schaft zum chirurgischen Werkzeug geleitet. Am Werkzeug und/oder am Handgriff sind häufig Getriebe zur Kraft- bzw. Momentenwandlung vorgesehen. In der Regel haben diese Getriebe aber kein lineares Übersetzungsverhältnis, was dazu führt, dass der Operateur unterschiedlich dicke Gewebe selbst bei gleicher Betätigungskraft mit einer anderen Kraft greift. Ferner machen auch Reibungsverluste, die im Getriebe und vor allem auch an Umlenkungen von Bowdenzügen auftreten, das Instrument schwergängig, was zu ermüdendem Arbeiten führen kann.

Daher wurden mikrochirurgische Instrumente mit hydraulischer Betätigung entwickelt, die geringere Verluste bei der Kraftübertragung von der Betätigungsvorrichtung zum Werkzeug aufweisen als die mit mechanischer Übertragung.

So beschreibt etwa die DE 195 37 897 A1 ein endoskopisches Instrument, an dessen distalem Ende eines Schafts verschiedene Chirurgiewerkzeuge angeordnet sind. Die einzelnen Chirurgiewerkzeuge können dort durch einen hydraulischen Aktor in eine Benutzungsposition gebracht werden, während die anderen sich in Ruhestellung befinden. Der hydraulische Aktor, etwa eine Kolben-Zylinder-Anordnung, betätigt jedoch das Chirurgiewerkzeug nicht direkt, er bringt es nur in die Benutzungsstellung. Es müssen daher viele Leitungen durch den Schaft geführt werden, die die Baugröße des Instruments unnötig erhöhen.

Ferner offenbart die DE 41 36 861 C2 ein mikrochirurgisches Instrument, das über ein schwenkbar am distalen Ende des Schafts angeordnetes Chirurgiewerkzeug verfügt. Das Chirurgiewerkzeug ist mittels eines Handgriffs bedienbar, wobei die Kraft- bzw. Signalübertragung zum Bedienen des Chirurgiewerkzeugs über Seilzüge elektrisch, hydraulisch oder pneumatisch erfolgt. Die hydraulische Betätigung des Chirurgiewerkzeugs wird dort nicht näher beschrieben.

Weiter beschreibt die DE 27 59 488 C2 ein chirurgisches Instrument mit hydraulisch betätigtem Werkzeug gleicher Bauart, bei dem das Werkzeug über einen Hydraulikzylinder betätigt wird, dessen Kolben mit einem Umlenkhebel gekoppelt ist, der wiederum auf die Branchen des Werkzeugs wirkt. Der Hydraulikzylinder kann über einen Druckerzeugungszylinder im Handgriff mit Druck beaufschlagt werden. Das dort offenbarte Instrument weist eine Vielzahl von Teilen auf, die aufwändig und teuer hergestellt werden, und es besteht aufgrund der vielen hydraulischen Verbindungen und mitbewegten Kolbendichtungen jederzeit die Gefahr von Undichtigkeiten.

Schließlich offenbart auch die DE 35 23 022 A1 ein chirurgisches Instrument bekannter Bauart, bei dem eine Schere am äußersten Punkt des Schafts angelenkt ist, sodass sie sich entgegen der Einführrichtung des Schafts öffnen lässt. Die Schere wird betätigt, indem das Schaftinnere mittels der Handhabe gegen das Schaftäußere verschoben wird. Die Betätigung kann auch hydraulisch erfolgen, wird jedoch nicht näher erläutert.

Ferner ist aus der DE 20 2007 009 310 U1 ein chirurgisches Instrument offenbart, das an seinem distalen Ende Werkzeugelemente in Form von Branchen hat, die über einen Betätigungsabschnitt beweglich miteinander verbunden sind. Der Betätigungsabschnitt ist mit einer elastischen Kammer verbunden, die mit Fluid gefüllt werden kann. Durch Füllen der Kammer mit dem Fluid wird eine Kraft auf den Betätigungsabschnitt ausgeübt und die Branchen werden zueinander bewegt.

In der EP 2 392 270 A1 ist ebenfalls ein chirurgisches Werkzeug mit einem proximalen Kopplungsabschnitt beschrieben, der zwei hydraulisch betätigbare, schwenkbar verbundene Branchen aufweist. Weitere solcher chirurgischen Instrumente sind aus der US 4 848 338 A, US 8 241 228 B1 und GB 2 161 707 A bekannt.

Ausgehend von diesem Stand der Technik liegt der vorliegenden Erfindung die Aufgabe zu Grunde, ein verbessertes chirurgisches Werkzeug zu schaffen, das über einen möglichst großen Betätigungsbereich eine lineare Rückmeldung der Betätigungskraft ermöglicht und aus wenigen, günstig herstellbaren Einzelteilen besteht.

Diese Aufgabe wird durch ein chirurgisches Werkzeug mit den Merkmalen des unabhängigen Anspruchs 1 gelöst.

Ferner ergibt sich die Aufgabe, ein verbessertes mikrochirurgisches Instrument bereitzustellen, das einem Bediener ein besseres Force-Feedback und ermüdungsarmes Arbeiten ermöglicht.

Diese Aufgabe wird durch ein mikrochirurgisches Instrument mit den Merkmalen des Anspruchs 9 gelöst.

Bevorzugte Ausführungsbeispiele der Vorrichtungen werden jeweils durch die Unteransprüche beschrieben.

Das erfindungsgemäße chirurgische Werkzeug weist nach einer ersten Ausführungsform einen proximalen Kopplungsabschnitt auf, aus dem sich zwei hydraulisch betätigbare, schwenkbar verbundene Branchen erstrecken. Diese haben zumindest an ihren distalen Enden jeweils einen Wirkabschnitt und bilden im Kopplungsabschnitt eine Begrenzung für wenigstens einen Teil einer fluiddichten und -befüllbaren Kavität. Die Schwenkposition der Branchen und das Fluidvolumen in der Kavität sind operativ miteinander gekoppelt. Ferner weist das chirurgische Werkzeug im Kopplungsabschnitt ein Werkzeuggehäuse mit zumindest einer Ausnehmung auf. In der Ausnehmung sind die schwenkbar verbundenen Branchen mit einem Führungsabschnitt geführt, wobei das Werkzeuggehäuse mit den Branchen die Kavität bildet. Ferner kann am Werkzeuggehäuse ein Hydraulikanschluss angeordnet sein, der mit der Kavität fluidisch verbunden ist.

Die Bezeichnungen proximal und distal sind hierin in Bezug auf die Anbringung des chirurgischen Werkzeugs an einem Werkzeugschaft zu verstehen, wie bei gattungsmäßigen chirurgischen Werkzeugen gemäß dem Stand der Technik bekannt. Demnach wird das chirurgische Werkzeug distal angebracht und kann mit einer proximalen Handhabe bedient werden.

Die Fluidfüllmenge in der Kavität und die Schwenkposition der Branchen hängen nach vorbekannten und bestimmbaren Beziehungen voneinander ab, während die Schwenkposition der Branchen einfach durch Einstellen der Füllung und des Drucks in der Kavität bestimmt werden kann. Die Kraftübertragung vom Fluid in der Kavität auf die Branchen erfolgt direkt durch Druckeinwirkung auf die Branchen, ohne dass Hydraulikzylinder, Umlenkhebel oder andere Getriebe notwendig wären, da in einem Fluid die Druckkräfte stets in alle Raumrichtungen gleich wirken. Es treten bis auf den Druckverlust des Fluids beim Einströmen in die Kavität kaum Reibungsverluste auf.

Das erfindungsgemäße chirurgische Werkzeug kann daher mit deutlich geringeren Bedienkräften als bekannte chirurgische Werkzeuge dieser Art bedient werden und es ermöglicht auch ein deutlich besseres Force-Feedback als diese, die etwa über Kniehebelgelenke bedient werden.

Vorteilhaft ist das erfindungsgemäße chirurgische Werkzeug nur aus einer geringen Teileanzahl aufgebaut, wodurch es sehr günstig herstellbar ist. Die Branchen umschließen die fluidgefüllte Kavität direkt, wobei an den Dichtflächen der Branchen eine dichtende Oberflächenbeschichtung oder sonstige berührende Dichtungen angebracht sein können, um sicherzustellen, dass die Kavität fluiddicht abgeschlossen ist. Solche Dichtelemente beeinflussen den Kraftfluss jedoch nur unwesentlich, sie sollen lediglich Undichtigkeiten verhindern, die bei der Verwendung im menschlichen Körper unbedingt vermieden werden müssen. Jedoch kann vorteilhaft auch ein biologisch unbedenkliches bzw. steriles Fluid eingesetzt werden, so dass eine Undichtigkeit im Hydraulikkreis nicht zu einer Infektion bzw. Kontamination von umliegendem Gewebe führt.

Die Kavität wird oben und unten jeweils von dem Führungsabschnitt einer Branche begrenzt und an seitlichen sowie proximalen und distalen Begrenzungsflächen vom Werkzeuggehäuse. Oben und sind hierin die Raumrichtungen, die durch die Öffnungs- bzw. Schließrichtung der Branchen vorgegeben sind. Der Führungsabschnitt liegt dabei vorteilhaft an einem dem Wirkabschnitt der Branchen abgewandten Ende. Da zwischen den Branchen und dem Werkzeuggehäuse bei Bewegung der Branchen eine Relativbewegung stattfindet, müssen sie gegeneinander abgedichtet sein. Dies kann beispielsweise durch eine Beschichtung auf der der inneren der Kavität zugewandten Oberfläche des Werkzeuggehäuses erfolgen oder beispielsweise durch einen O-Ring, der in einer Aufnahmenut der Mantelfläche der Führungsabschnitte angeordnet ist. Um eine langlebige Abdichtung zu erreichen, sollten die Führungsabschnitte entlang der Aufnahmenut verrundete Kanten aufweisen.

In einer weiteren Ausführungsform können die Branchen im Werkzeuggehäuse schwenkbar auf einer Führungsachse gelagert sein und die Ausnehmung kann in der Schwenkebene der Branchen liegen.

Die Führungsachse kann sich vorteilhaft zwischen dem Führungsabschnitt und dem Wirkabschnitt der Branchen erstrecken, so dass eine auf den Führungsabschnitt aufgebrachte Kraft ein Moment um die Führungsachse erzeugt, das zur Betätigung der Branchen führt. Vorteilhaft wird durch die Ausrichtung der Ausnehmung parallel zur Schwenkebene der Branchen erreicht, dass an den Führungsabschnitten nur Kräfte angreifen, die in der Schwenkebene der Branchen wirken, während die Innenwandung des Werkzeuggehäuses die Kräfte, die parallel zur Schwenkachse der Branchen wirken, aufnimmt.

Des Weiteren kann der Hydraulikanschluss in einen Ballon münden, der in der Kavität angeordnet ist. Es ist vorteilhaft, wenn der Ballon mit den Branchen verbunden ist, und besonders vorteilhaft, wenn er mit den Branchen wenigstens punktuell verklebt ist.

Die Kavität wird zur leichteren Abdichtung mit dem Ballon, einer Art "Blase", ausgelegt, die sich bei Befüllung an die Begrenzungswände der Kavität anschmiegt. In diesem Fall kann beispielsweise auf Abdichtungsmaßnahmen zwischen den Führungsabschnitten der Branchen und dem Werkzeuggehäuse verzichtet werden, es muss lediglich ein Höchstspaltmaß eingehalten werden, da ansonsten der Ballon bei Druckbeaufschlagung in den Spalt rutschen könnte. Gemäß dieser Ausführungsform ist es möglich, das chirurgische Werkzeug noch günstiger herzustellen, da viel geringere Toleranzen eingehalten werden müssen. Indem der Ballon mit den Führungsabschnitten verklebt wird ist es möglich, nicht nur Druckkräfte auf die Branchen auszuüben, sondern auch Zugkräfte beim Entleeren bzw. Zusammenschrumpfen des Ballons, wodurch auf zusätzliche Rückholeinrichtungen verzichtet werden kann. Jedoch soll die Wandstärke des Ballons nicht zu groß sein, da ansonsten der zum Ausdehnen notwendige Druck zu groß wird, was, wenn keine weiteren Vorkehrungen getroffen werden, die Force-Feedback-Eigenschaften verschlechtern kann, da stets die Rückstellkraft des Ballons auf das Fluid wirkt; es sei denn, eine Betätigungsvorrichtung zur Druckbeaufschlagung des Ballons werden mit der gleichen Kraft vorgespannt wie der Ballon, so dass sich die jeweiligen Rückstellkräfte gerade kompensieren würden.

Alternativ kann ein elektrisches Stellglied zum Einstellen des Drucks und der Füllung der Kavität fluidisch mit der Kavität verbunden sein. Vorteilhaft handelt es sich bei dem Stellglied um eine Pumpe, die mit dem Hydraulikanschluss verbunden ist. Die Pumpe kann ferner am Werkzeuggehäuse angeordnet sein und kann vorteilhaft mit einem Ausgleichsbehälter, der auch am Werkzeuggehäuse angeordnet sein kann, fluidisch verbunden sein.

Miniaturiserte Pumpen, beispielsweise Membranpumpen in Streichholzkopfgröße, sind Stand der Technik, so dass diese Ausführungsform die Gesamtbaugröße des erfindungsgemäßen chirurgischen Werkzeugs nur unwesentlich erhöht.

Gemäß einer weiteren Ausführungsform kann das chirurgische Werkzeug eine Rückstellvorrichtung aufweisen, mit der die Branchen in ihre Ruhestellung rückgestellt werden können. Bei der Rückstellvorrichtung kann es sich vorteilhaft um einen Federring oder einen Elastomerring handeln, der besonders vorteilhaft kraftleitend mit dem Führungsabschnitt der Branchen verbunden ist. Bei der Ruhestellung sind die Brachen vorzugsweise geöffnet.

Die Rückstellvorrichtung kann zur Rückstellung der Branchen Druck- oder Zugkräfte ausüben, das bedeutet, sie kann die Branchen entweder von innen in die Ruhstellung "drücken" oder aber von außen angreifen. Handelt es sich bei der Rückstellvorrichtung um einen von außen auf die Führungsabschnitte der Brachen gesetzten Feder- oder Elastomerring, so ist es vorteilhaft, wenn er in einer außenliegenden Aufnahmenut der Führungsabschnitte aufgenommen ist, sodass er nicht unbeabsichtigt herunterrutschen kann. Bei der Auswahl des Federrings ist jedoch darauf zu achten, dass die ausgeübte Rückstellkraft gerade ausreicht, um die Reibung der schwenkbaren Anlenkung und den Druckverlust des Fluids beim Ausströmen aus der Kavität zu überwinden, da ansonsten die Force-Feedback-Eigenschaften auch hierdurch beeinträchtigt werden können. Dies kann jedoch verhindert werden, indem die Betätigungsvorrichtung, beispielsweise an einem Handgriff, zusätzlich um den Betrag der Rückstellkraft des Federrings vorgespannt wird, wodurch sich die beiden Kräfte aufheben und ein sehr feinfühliges Force-Feedback ermöglicht wird.

Um zu verhindern, dass Gewebe durch zu starke Krafteinwirkung geschädigt wird, kann das mikrochirurgische Instrument auch ein Überdruckventil aufweisen, das bei einem vorbestimmten Auslösedruck öffnet und beispielsweise an eine Rücklaufleitung angeschlossen ist. Auf die Rücklaufleitung kann jedoch verzichtet werden, wenn es sich bei dem Hydraulikfluid um ein biokompatibles Fluid handelt.

In ihren Wirkabschnitten können die Branchen gemäß einer weiteren Ausführungsform Schneiden oder Greifflächen aufweisen. Es können hierbei sämtliche Arten bekannter Greifflächen zum Einsatz kommen, insbesondere spezielle Nadelgreifer oder atraumatisch gestaltete Greifflächen, die zum Greifen von empfindlichem Gewebe geeignet sind.

Das chirurgische Werkzeug kann wie folgt betätigt werden:
- Befüllen der Kavität mit einem vorbestimmten Fluidvolumen, dadurch Vergrößern des Volumens der Kavität und zwangsgekoppelt Bewegen der Branchen bis zu einer vorbestimmten Schwenkposition, und/oder
- Einstellen eines vorbestimmten Drucks in der Kavität, dadurch Übertragen einer Druckkraft von der Kavität auf die Branchen und Ausüben einer vorbestimmten Betätigungskraft der Branchen.

Es kann dabei ein kompressibles oder ein inkompressibles Fluid zum Einsatz kommen. Wenn ein inkompressibles Fluid verwendet wird, so kann keine Bewegung der Branchen ohne Änderung des Fluidvolumens der Kavität stattfinden; die Änderung der Fluidmenge hängt nur von der Kinematik und Geometrie der Branchen bzw. der Kavität ab. Anders, wenn ein kompressibles Fluid zum Einsatz kommen soll: In diesem Fall hängt das der Kavität für eine Änderung der Schwenkposition zu- oder abzuführende Fluidvolumen auch noch von den Kräften auf die Branchen und damit vom Druck in der Kavität ab. In der Regel wird aber ein inkompressibles Fluid verwendet werden, da damit eine bessere Kraftübertragung erzielt werden kann und Undichtigkeiten im hydraulischen System keine plötzliche Expansion des Fluids zur Folge haben.

Eine erste Ausführungsform des mikrochirurgischen Instruments weist eine proximale Handhabe mit einer Betätigungsvorrichtung und ein distal angeordnetes erfindungsgemäßes, hydraulisch betätigbares chirurgisches Werkzeug auf. Das chirurgische Werkzeug ist durch einen Schaft mit der Handhabe verbunden, während der Druck und die Füllung der Kavität des chirurgischen Werkzeugs mittels der Betätigungsvorrichtung einstellbar sind.

Bei der Handhabe bzw. der Kombination aus Handhabe und Betätigungsvorrichtung kann es sich beispielsweise um einen Pinzettengriff, einen Zangengriff, einen Pistolengriff oder eine Ringsteuerung handeln. Die Kopplung zur Übertragung der Betätigungsparameter zum chirurgischen Werkzeug kann mechanisch, hydraulisch, elektrisch oder gar optisch erfolgen. Das Übersetzungsverhältnis zwischen der Betätigungsvorrichtung und der Bewegung der Branchen des chirurgischen Werkzeugs kann konstant oder verstellbar sein, wobei die Verstellbarkeit des Übersetzungsverhältnisses den Anforderungen während einer Operation entgegenkommt. Es muss bei einer Operation häufig zwischen Tätigkeiten, die verschiedene Sensibilität verlangen, abgewechselt werden, beispielsweise dem Greifen, Präparieren und/oder Durchtrennen von unterschiedlichen Gewebe-Typen und dem Halten einer Nadel, wobei jede dieser Tätigkeiten unterschiedliche Bedienkräfte des chirurgischen Werkzeugs erfordert.

In einer weiteren Ausführungsform des mikrochirurgischen Instruments kann das chirurgische Werkzeug dauerhaft oder auswechselbar mit dem Schaft verbunden sein. Die Handhabe kann vorteilhaft eine Rückstellvorrichtung zur Rückstellung der Betätigungsvorrichtung in die Ruhestellung aufweisen.

Unter Rückstellvorrichtung können hier federnde Bauteile verstanden werden, die bei Betätigung eine Rückstellkraft auf die Bedienvorrichtung ausüben oder aber eine Bedienvorrichtung, die Bedienflächen zur bidirektionalen Bedienung aufweist, beispielsweise ähnlich des Eingriffsauges einer Schere, sodass ein Operateur das chirurgische Werkzeug aktiv sowohl schließen als auch öffnen kann.

Gemäß einer weiteren Ausführungsform kann an der Handhabe eine Druckerzeugungsvorrichtung angeordnet sein, die mit der Betätigungsvorrichtung betätigt werden kann. Die Druckerzeugungsvorrichtung ist mit einer Fluidführungsvorrichtung, etwa einem Rohr oder einem Schlauch, fluidisch mit der Kavität des chirurgischen Werkzeugs verbunden, bevorzugt mit dem Hydraulikanschluss verbunden. Ferner kann es sich bei der Druckerzeugungsvorrichtung vorteilhaft um einen fluidgefüllten Ballon handeln, der mit der Betätigungsvorrichtung komprimierbar ist.

Der Ballon und die Kavität bilden dabei ein geschlossenes hydraulisches System, d. h., wenn der Ballon der Druckerzeugungsvorrichtung zusammengedrückt wird, wird Fluid aus dem Ballon in die Kavität gepumpt, wodurch sich ihr Volumen vergrößert und die Branchen des chirurgischen Werkzeugs betätigt werden. Die Verwendung eines Ballons ist eine sehr günstige Lösung, deutlich günstiger als ein hydraulischer Geberzylinder. Der Durchmesser der Fluidführungsvorrichtung darf nicht zu klein und/oder die Rauigkeit nicht zu groß gewählt werden, da ansonsten aufgrund erhöhter Strömungsgeschwindigkeit in der Fluidführungsvorrichtung ein größerer Druckverlust auftritt, der es wiederum schwierig macht, das mikrochirurgische Instrument präzise zu bedienen und auch die Kraftrückmeldung während einer Bewegung des chirurgischen Werkzeugs verfälschen kann.

Alternativ kann an der Handhabe zumindest ein elektrisches Messglied angeordnet sein, mit dem wenigstens die Betätigungskraft der der Betätigungsvorrichtung gemessen werden kann. Vorteilhaft ist zusätzlich zur Betätigungskraft auch der Betätigungsweg messbar. Das elektrische Messglied ist operativ mit dem elektrischen Stellglied des chirurgischen Werkzeugs verbunden.

Es sind in dieser Ausführungsform keine hydraulischen Leitungen durch den Schaft nötig, wodurch der Schaft mit einem geringeren Durchmesser gebaut werden kann. Das Messglied ist direkt oder indirekt mit dem Stellglied des chirurgischen Werkzeugs verbunden. Messglieder zur Messung von Kraft und/oder Weg sind bekannt und auch in miniaturisierter Bauweise erhältlich. Die gemessenen Betätigungsparameter, etwa Weg und/oder Kraft, können durch geeignete zwischengeschaltete elektrische bzw. elektronische Schaltungen zur Anpassung des Übersetzungsverhältnisses gewandelt werden. Es ist auch denkbar, dass ein Mikroprozessor sowie ein Speicher dazwischengeschaltet sind, um vorbestimmte Übersetzungsprofile mittels einer Bedienvorrichtung abrufen zu können. Um Force Feedback zu ermöglichen, können auch Stellmotoren an der Handhabe angeordnet sein, die proportional zur Betätigungskraft der Branchen auf die Betätigungsvorrichtung einwirken.

Gemäß einer weiteren Ausführungsform kann im Schaft eine Schubstange längsaxial verschiebbar gelagert sein, die mechanisch mit der Betätigungsvorrichtung gekoppelt ist und sich bis zu dem chirurgischen Werkzeug erstreckt. Die Schubstange taucht bei einer Betätigung der Betätigungsvorrichtung entlang eines Längenabschnitts in die Kavität ein.

Die Betätigung des chirurgischen Werkzeugs erfolgt dabei weiterhin hydraulisch, während die Signal- bzw. Kraftübertragung zur Auslösung des hydraulischen Betätigungsmechanismus' mechanisch erfolgt. Indem die Schubstange in die Kavität eintaucht, wird eine Verdrängung des Fluids in die einzig mögliche Ausdehnungsrichtung erreicht und sich die Branchen bewegen. Die Schubstange bildet in ihrem Endabschnitt, der in die Kavität eintaucht, quasi einen Betätigungskolben. Die Abdichtung ist unkritisch und kann über berührende Dichtungen erfolgen oder es kann ganz auf eine zusätzliche Abdichtung verzichtet werden, wenn die Kavität mit dem Ballon ausgekleidet ist.

Darüber hinaus kann der Schaft wenigstens entlang eines Längenabschnitts biegsam und/oder starr sein. Zusätzlich oder alternativ kann der Schaft längenverstellbar sein, wobei ein teleskopierbarer Schaft vorteilhaft ist. Der Schaft kann auch wenigstens ein Gelenk aufweisen, wobei ein Gelenk mit zwei Rotationsfreiheitsgraden bevorzugt wird.

Der Schaft-Typ kann gemäß den Anforderungen des Operations-Typs ausgewählt werden. Der typischen Bauart laparoskopsicher Instrumente entsprechend, kann er komplett starr sein und würde der Einfachheit wegen aus einem Rohr bestehen, in dem entweder ein Schlauch geführt sein kann oder das selbst die Fluidführungsvorrichtung bildet. Alternativ kann auch vorgesehen sein, dass ein an sich starrer Schaft an bestimmten Stellen Schwenkgelenke aufweist. Selbst durch den Gelenkbereich kann problemlos ein Schlauch geführt werden, während bei bekannten mechanischen Lösungen pro Gelenk ein teures, anfälliges und reibungsbehaftetes Übertragungsgelenk der Schubstange nötig wäre. Es kann sogar vorgesehen sein, einzelne Gelenke durch Stellvorrichtungen gesteuert zu bewegen, sodass die Ausrichtung des chirurgischen Werkzeugs am distalen Ende des Schafts beispielsweise durch eine Bedienvorrichtung an der Handhabe gesteuert werden kann. Ein flexibler Schaft kann etwa durch einen Schwanenhals-Aufbau realisiert werden und bietet in Kombination mit der hydraulischen oder elektrischen Bedienung besondere Vorteile: Im Gegensatz zu bekannten bowdenzug-betätigten Systemen ist insbesondere bei eng gebogenem Schaft keine Veränderung der Betätigungscharakteristik feststellbar.

Das erfindungsgemäße mikrochirurgische Instrument kann wie folgt betätigt werden:
- Betätigen der Betätigungsvorrichtung mit wenigstens einer vorbestimmten Betätigungskraft,
- aus der vorbestimmten Betätigungskraft nach einem vorbestimmen Übersetzungsverhältnis Bestimmen und Einstellen des Drucks der Kavität des chirurgischen Werkzeugs,
- dadurch Übertragen einer Druckkraft von der Kavität auf die Branchen und Ausüben einer vorbestimmten Betätigungskraft der Branchen.

Die Branchen des chirurgischen Werkzeugs werden in diesem Fall nicht oder nur unwesentlich bewegt, was voraussetzt, dass ein steifer Körper, beispielsweise eine Nadel, zwischen den Branchen angeordnet ist.

Ferner kann die Betätigungsvorrichtung mit einer vorbestimmen Betätigungskraft und einem vorbestimmten Betätigungsweg betätigt werden:
- aus dem vorbestimmten Betätigungsweg nach dem vorbestimmen Übersetzungsverhältnis Bestimmen und Einstellen der Füllung der Kavität des chirurgischen Werkzeugs,
- dadurch zwangsgekoppelt Bewegen der Branchen bis zu einer vorbestimmten Schwenkposition.

Das vorbestimmte Übersetzungsverhältnis für die Kraft und/oder Wegübertragung kann durch eine Abstimmung der Kinematik des chirurgischen Werkzeugs, der Flächen auf die der Fluiddruck einwirkt und/oder der Kinematik der Betätigungsvorrichtung erreicht werden. Bei elektrischer bzw. elektronischer Signalübertragung kann eine solche Abstimmung noch einfacher erfolgen, ohne dass Änderungen am mechanischen und/oder hydraulischen System vorzunehmen wären.

Diese und weitere Vorteile werden durch die nachfolgende Beschreibung unter Bezug auf die begleitenden Figuren dargelegt. Der Bezug auf die Figuren dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich schematische Darstellungen von Ausführungsbeispielen der Erfindung. Es zeigen:

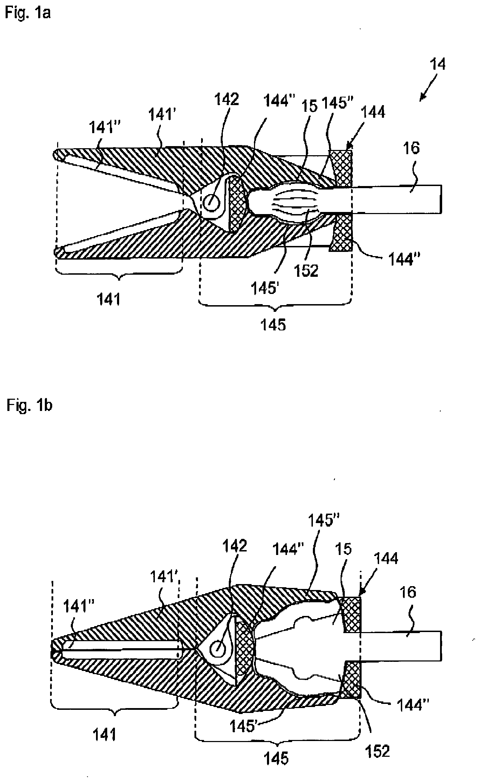

- **Fig. 1a**: eine Schnittansicht des chirurgischen Werkzeugs mit geöffneten Branchen,
- **Fig. 1**: **b** eine Schnittansicht des chirurgischen Werkzeugs mit geschlossenen Branchen,
- **Fig. 2a**: eine perspektivische Ansicht einer Branche des chirurgischen Werkzeugs,
- **Fig. 2b**: eine perspektivische Ansicht einer Branche und des Werkzeuggehäuses des chirurgischen Werkzeugs,
- **Fig. 3**: eine perspektivische Ansicht des chirurgischen Werkzeugs mit Rückstellvorrichtung,
- **Fig. 4**: eine perspektivische Ansicht des mikrochirurgischen Instruments.

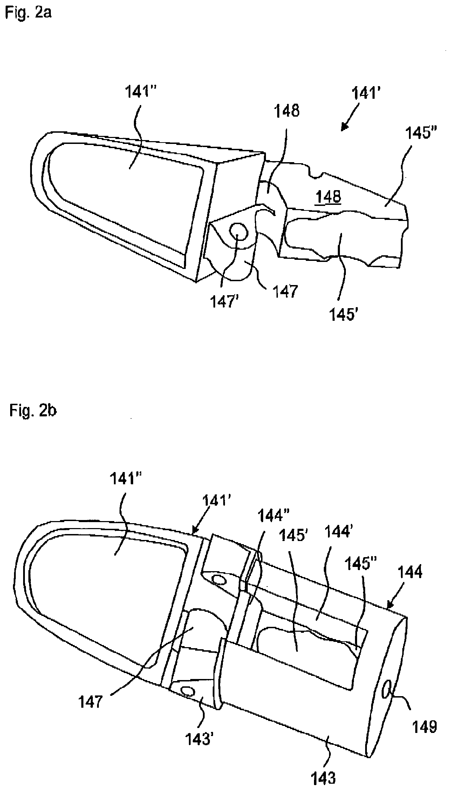

In **Fig. 1a** ist ein Längsschnitt des chirurgischen Werkzeugs 14 durch die Mittelebene dargestellt. Es weist zwei schwenkbar auf einer Führungsachse 142 gelagerte Branchen 141' auf, wobei die Führungsachse 142 in einem Werkzeuggehäuse 144 gelagert ist. Mit dem Kopplungsabschnitt 145, genauer mit den Außenflächen des Werkzeuggehäuses 144, kann das chirurgische Werkzeug 14 mit einem Schaft eines mikrochirurgischen Instruments gekoppelt werden, um in der minimalinvasiven Chirurgie eingesetzt zu werden. In ihrem Wirkabschnitt 141 tragen die Branchen 141' Greifflächen 141", wobei es auch möglich ist, aber nicht dargestellt, dass im Wirkabschnitt 141 andere Wirkflächen, wie beispielsweise Schneiden, vorliegen. Am anderen Ende der Branchen 141', das dem Wirkabschnitt 141 abgewandt ist, dem Führungsabschnitt 145", umschließen die Branchen 141' eine fluidgefüllte Kavität 15, deren Volumen sich in Abhängigkeit der Schwenkposition der Branchen 141' verändert. Die Führungsabschnitte 145" sind in einer Ausnehmung 144' (siehe **Fig. 2b**) des Werkzeuggehäuses 144 geführt, sodass die Kavität 15 oben und unten jeweils von den Führungsabschnitten 145" begrenzt wird und seitlich von den Wänden der Ausnehmung 144' (siehe dazu **Fig. 2b**).

Die Kavität 15 ist zur besseren Abdichtung mit einem Ballon 152 ausgekleidet, der fluidisch mit einer Fluidführungsvorrichtung 16 verbunden ist. Es ist jedoch auch eine Ausführungsform ohne den Ballon 152 möglich, jedoch sind die Anforderungen an die Maßhaltigkeit und Oberflächengüte der Dichtflächen 148,144" (siehe auch **Fig. 2a**) in diesem Fall sehr hoch; kleinster Verschleiß der Dichtflächen 148,144" könnte zu Undichtigkeiten führen, was bei der Verwendung des chirurgischen Werkzeugs 14 im menschlichen Körper ausgeschlossen sein muss. Die Wandstärke des Ballons 152 darf jedoch nicht zu groß sein, da ansonsten seine Rückstellkraft bei der Befüllung zu groß wäre. In diesem Fall wäre eine genaue Rückmeldung der Betätigungskraft nicht möglich, da der Druck in der Kavität 15 zu einem maßgeblichen Teil durch die Rückstellkraft des Ballons 152 bestimmt würde. Daher sollte die Wandstärke des Ballons 152 gerade so dick gewählt werden, dass er dem maximal auftretenden Betriebsdruck widerstehen kann und hinreichend robust gegen mechanische Beschädigung ist. Um eine Beschädigung des Ballons 152 zu verhindern sind die Kanten der Aushöhlungen 145' der Branchen 141', die die Kavität 15 mit bilden, zudem verrundet. Damit die Branchen 141' möglichst weit geöffnet werden können, sind die Aushöhlungen 145' groß genug, dass der leere Ballon 152 vollständig darin aufgenommen werden kann.

Durch die Fluidführungsvorrichtung 16 kann die Kavität 15 bzw. der Ballon 152 mit Fluid befüllt oder entleert werden. Wird die Füllung erhöht, legt sich der Ballon 152 zunächst an die Aushöhlungen 145' der Branchen 141 an, dann schließen sich die Branchen 141' infolge einer Kraftausübung vom Ballon 152 auf die Führungsabschnitte 145" Branchen 141' und des resultierenden Moments um den Drehpunkt der Führungsachse 142.

Der Zustand mit geschlossenen Branchen 141' und gefüllten Ballon 152 ist in der Schnittansicht der **Fig. 1b** dargestellt. Der Ballon 152 schmiegt sich hier vollständig an die Oberflächen der Aushöhlungen 145' an. Die Konturen der Aushöhlungen 145' in der Schnittebene zeichnen sich auf dem Ballon 152 ab, da die Aushöhlungen 145' normal zur Bewegungsebene der Branchen 141' einen veränderlichen Querschnitt aufweisen. Bei Befüllung des Ballons 152 kann er sich nach oben/unten nur in die jeweilige Schwenkrichtung der Branchen 141 ausdehnen, während seine Ausdehnung zum proximalen und distalen Ende durch Begrenzungsabschnitte 144" des Werkzeuggehäuses 144 verhindert wird. Bei einer Befüllung und/oder Druckänderung des Ballons 152 wird die Kraft direkt auf die Branchen 141' ausgeübt, ohne dass die Kraft durch weitere Umlenkhebel o. ä, noch umgelenkt werden müsste.

Das erfindungsgemäße chirurgische Werkzeug 14 zeichnet sich aufgrund dieser Bauweise durch eine deutlich geringere Teileanzahl als bekannte chirurgische Werkzeuge mit hydraulischer Bedienung aus, da auf Hydraulikzylinder und/oder weitere Einrichtungen zur Kraftwandlung verzichtet werden kann. Dies führt dazu, dass das chirurgische Werkzeug 14 vergleichweise günstig herstellbar ist. Zur günstigen Herstellbarkeit trägt ferner die Auskleidung der Kavität 15 mit dem Ballon 152 bei, da deutlich geringere Anforderungen an die Maßhaltigkeit und/oder Oberflächengüte der Dichtflächen 148,144" gestellt werden, als bei einer Ausführungsform ohne Ballon 152. Außerdem sind zur Bedienung viel niedrigere Bedienkräfte nötig, da keine Reibung in Getrieben und/oder Gelenken, wie bei bekannten mechanischen chirurgischen Werkzeugen, auftritt.

In **Fig. 2a** ist eine Branche des chirurgischen Werkzeugs 14 perspektivisch dargestellt, wobei die verrundete Form der Aushöhlung 145' in der Branche 141', die die Kavität 15 (siehe **Fig. 1a**) mit bildet, gut zu erkennen ist. Die Aushöhlung 145' ist so geformt, dass der leere Ballon 152 (siehe **Fig. 1b**) bei geöffneten Branchen 141' darin aufgenommen werden kann. Die Branche 141' verfügt über einen Scharnierabschnitt 147, in dem eine Durchgangsbohrung 147' vorliegt, durch die die Führungsachse 142 (siehe **Fig. 2b**) gesteckt wird. Der proximale Bereich "rechts" des Scharnierabschnitts 147 ist der Führungsabschnitt 145" der Branche 141', der so gestaltet ist, dass er in der Ausnehmung 144' des Werkzeuggehäuses 144 (siehe **Fig. 2b**) so geführt werden kann, dass die Dichtflächen 148 und die Ausnehmung 144' des Werkzeuggehäuses 144 entweder direkt eine fluiddichte Abdichtung der Kavität 15 ermöglichen oder nur einen schmalen Spalt freigeben, so dass der Ballon 152 (siehe **Fig. 1b**) nicht in den Spalt gedrückt werden kann. Ferner ist die Greiffläche 141" der Branche 141' zu sehen, mit der beispielsweise Tupfer, Nadeln und/oder Gewebe gehalten werden können

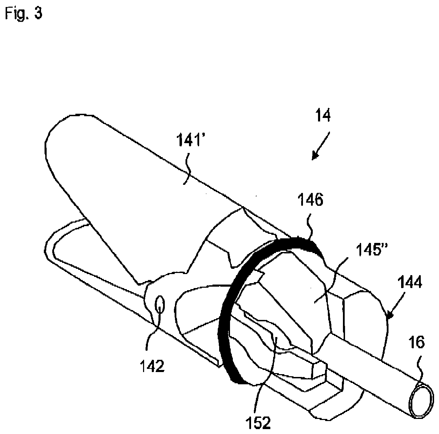

**Fig. 2b** zeigt das Werkzeuggehäuse 144 und die andere Branche 141' des chirurgischen Werkzeugs 14 (siehe **Fig. 3**), wobei auch die andere Branche 141' einen Scharnierabschnitt 147 mit einer Durchgangsbohrung für die Führungsachse 142 aufweist. Die Führungsachse 142 (siehe Fig. 1a) wird zum Zusammenbau des chirurgischen Werkzeugs 14 durch die Durchgangsbohrungen der jeweiligen Scharnierabschnitte 147 der Branchen 141' sowie durch die Anbindungsabschnitte 143' des Werkzeuggehäuses 144 gesteckt. Die Ausnehmung 144' weist neben zwei seitlichen Wänden proximale und distale Begrenzungsabschnitte 144" auf, die in Paarung mit den Dichtflächen 148 der Führungsabschnitte 145" der Branchen 141' die Abdichtung der Kavität ermöglichen. Um die Abdichtung der Kavität zu verbessern, können die Wände und/oder die Begrenzungsabschnitte 144" der Ausnehmung 144' mit einer Kunststoffschicht beschichtet sein oder die Kavität kann mit einem Ballon 152 ausgekleidet sein (siehe **Fig. 3**). An der proximalen Stirnfläche des Werkzeuggehäuses 144 liegt ein Hydraulikanschluss 149 vor, der fluidisch mit der Kavität verbunden ist. Kommt ein Ballon 152 zum Einsatz, so ist es für die Montage des chirurgischen Werkzeugs vorteilhaft, wenn der Ballon in leerem Zustand durch den Hydraulikanschluss 149 in die Kavität geschoben werden kann.

Die perspektivische Ansicht des zusammengebauten chirurgischen Werkzeugs 14 der **Fig. 3** zeigt zusätzlich eine Rückstellvorrichtung 146, die umlaufend um das Werkzeuggehäuse 144 und die Branchen 141' angeordnet ist. Es handelt sich bei der Rückstellvorrichtung 146 um einen Federring oder Elastomerring, der in der dargestellten geöffneten Position der Branchen 141' entspannt ist. Werden die Branchen 141' an ihrem distalen Ende geschlossen, übt der Ring der Rückstellvorrichtung 146 eine Rückstellkraft auf den proximalen Führungsabschnitt 145" der Branchen aus. Der Ring der Rückstellvorrichtung 146 ist in korrespondierende Aufnahmenuten der Branchen 141' eingelegt, sodass er nicht unbeabsichtigt herunterrutschen kann. In dieser Darstellung ist gut zu sehen, dass in der Kavität zwischen den Führungsabschnitten 145 der beiden Branchen 141' der Ballon 152 aufgenommen ist, der hier leer ist. Der Ballon 152 ist fluidisch mit einem Rohr oder einen Schlauch 16 verbunden, über den der Ballon 152 befüllt/entleert und/oder mit Druck beaufschlagt werden kann. Zusätzlich kann über das Werkzeuggehäuse 144 und einen proximalen Längenabschnitt der Branchen 141 eine Abdeckung geschoben sein, die die Rückstellvorrichtung 146 verdeckt und es verhindert, dass Verunreinigungen eindringen können, was figurativ nicht gezeigt ist. Die Federsteifigkeit des Rings der Rückstellvorrichtung 146 sollte dabei gerade so hoch gewählt werden, dass die Rückstellkraft bei geschlossenen Branchen 141 gerade ausreicht, um die Branchen 141 zu öffnen, darf jedoch auch nicht zu hoch sein, da ansonsten die Kraftrückmeldung an den Operateur verfälscht würde.

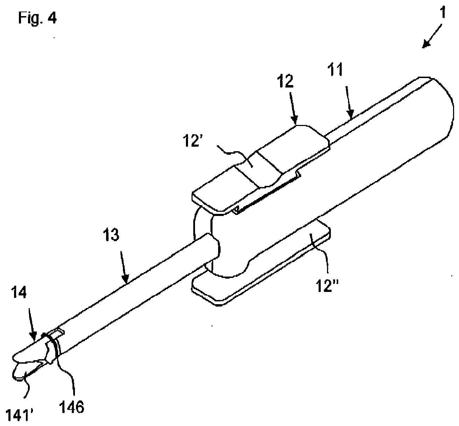

**Fig. 4** zeigt das erfindungsgemäße mikrochirurgische Werkzeug 1, das eine Handhabe 11 und ein über einen Schaft 13 angebundenes chirurgisches Werkzeug 14 mit zwei schwenkbaren Branchen 141' aufweist. An der Handhabe 11 ist eine Betätigungsvorrichtung 12 angeordnet, mit der das chirurgische Werkzeug 12 bedienbar ist. Im Inneren der Handhabe 11 befindet sich ein zweiter Ballon, der als Druckerzeugungsvorrichtung dient. Dieser zweite Ballon ist in einem geschlossenen hydraulischen Kreis fluidisch mit dem Ballon in der Kavität des chirurgischen Werkzeugs 14 verbunden und wird bei Betätigung der Betätigungsvorrichtung 12 zusammengedrückt. Zur hydraulischen Verbindung der beiden Ballone ist im Schaft 13 ein Rohr oder Schlauch 16 (siehe **Fig. 3**) geführt, der idealerweise sehr druckfest ist, um zu verhindern, dass durch Querdehnung des Schlauchs 16 die Kraftrückmeldung behindert wird. Hier kann etwa ein gewebeummantelter Schlauch eingesetzt werden.

Es kann auch vorgesehen sein, dass zwei Schläuche im Schaft 13 geführt sind; ein Schlauch 16 zur Druckversorgung und ein anderer zur Rückführung von Fluid, das an einem Überdruckventil am mikrochirurgischen Werkzeug ausgetreten ist und zurück in ein Reservoir geführt werden muss. Alternativ kann das Überdruckventil aber auch an der Handhabe angeordnet sein; so kann auf den Rückführschlauch verzichtet werden, was jedoch in den Figuren nicht gezeigt ist.

Die Betätigungsvorrichtung 12 besteht aus zwei beweglichen Platten 12", die im Inneren der Handhabe 11 schwenkbar angelenkt sind; sie bildet also quasi einen Pinzettengriff, der mit den Fingerspitzen von Daumen und Zeigefinger bedient werden kann. Die Platten 12" weisen jeweils eine rinnenförmige Ausnehmung 12' auf, die erstens der Ergonomie dient, da die Fingerkuppen bequem darauf gesetzt werden können, und zweitens die Position auf den Platten 12" markiert, an der der Betätigungsweg und die Betätigungskraft der Betätigungsvorrichtung genau dem Weg und der Kraft an den Branchen 141' entsprechen. Hierzu können die Platten 12" der Betätigungsvorrichtung innen liegend jeweils einen Stempel aufweisen, der auf den Betätigungsballon drückt; die Krafteinleitung von den Platten 12" in den Ballon erfolgt somit immer an der gleichen Stelle, egal an welcher Längsposition der Platten 12" der Operateur seine Fingerkuppen auch positioniert hat. Jedoch ist bei einer Veränderung der längsaxialen Krafteinleitungsposition auf den Platten eine Änderung des Übersetzungsverhältnisses erreichbar; da die Platten 12" schwenkbar angelenkt sind führt ein "Eindrücken" der Platten 12" um einen festen Betätigungsweg in Abhängigkeit der Krafteinleitungsposition zu einem unterschiedlichen Weg der Branchen. Ein Operateur kann diese "Hebel-Charakteristik" gezielt nutzen und beispielsweise zum Halten von Nadeln eine Krafteinleitungsposition wählen, die ihm bei einem großen Betätigungsweg und einer kleinen Betätigungskraft eine große Kraft und einen kleinen Weg der Branchen ermöglicht. Der Operateur kann jederzeit durch Verschieben seiner Fingerspitzen zum "ausgeglichenen" Übersetzungsverhältnis zurückkehren, das ihm eine direkte Rückmeldung der Kraft an den Branchen erlaubt, indem er einfach die rinnenförmige Ausnehmung 12' ertastet.

An der Handhabe 11 kann zusätzlich auch eine Sperrvorrichtung angeordnet sein, mit der die Betätigungsparameter der Betätigungsvorrichtung 12 arretiert werden können, was jedoch nicht figurativ gezeigt ist. Dabei kann es sich etwa um einen Schieber handeln, der mit der Handhabe 11 verbunden ist und an dessen Ende eine Ratnase angeordnet ist. Die Rastnase kann zum Arretieren der Schwenkposition in Eingriff mit einem diskreten Rastrelief der Betätigungsvorrichtung 12 gebracht werden. Alternativ kann die Sperrvorrichtung die Betätigungsvorrichtung 12 auch kraftschlüssig arretieren, was eine stufenlose Arretierung ermöglicht.

Zur Rückstellung des chirurgischen Werkzeugs 14 vom geschlossenen in den geöffneten Zustand ist, wie schon in **Fig. 3** dargestellt, ein Federring 146 kraftleitend umlaufend um die Branchen 141' angeordnet.

Es ist jedoch auch eine Ausführungsform ohne separate Rückstellvorrichtung 146 am chirurgischen Werkzeug 14 denkbar, die jedoch in der **Fig. 4** nicht gezeigt ist. Der Ballon in der Kavität des chirurgischen Werkzeugs 14 kann die Branchen bei Druckbeaufschlagung nicht nur schließen, sondern diese auch wieder öffnen, indem der Ballon die Führungsabschnitte der Branchen 141 bei Kontraktion aktiv mitnimmt; dazu müsste der Ballon jedoch zumindest punktuell mit den Führungsabschnitten der Branchen 141 verbunden sein, beispielsweise verklebt.

## Patentansprüche

1. Chirurgisches Werkzeug (14),
das einen proximalen Kopplungsabschnitt (145) aufweist, aus dem sich zwei hydraulisch betätigbare, schwenkbar verbundene Branchen (141') erstrecken, die zumindest an ihren distalen Enden jeweils einen Wirkabschnitt (141) haben,
**wobei**
die Branchen (141') in dem Kopplungsabschnitt (145) eine Begrenzung für zumindest einen Teil einer fluiddichten, fluidbefüllbaren Kavität (15) bilden, wobei eine Schwenkposition der Branchen (141') und ein Volumen des Fluids in der Kavität (15) operativ miteinander gekoppelt sind,
**dadurch gekennzeichnet, dass**
das chirurgische Werkzeug (14) in dem Kopplungsabschnitt (145) ein Werkzeuggehäuse (144) mit zumindest einer Ausnehmung (144') aufweist, in der die schwenkbar verbundenen Branchen (141') mit einem Führungsabschnitt (145") geführt sind, wobei an dem Werkzeuggehäuse (144) ein Hydraulikanschluss (149) angeordnet ist und wobei das Werkzeuggehäuse (144) mit den Branchen (141') die Kavität (15) bildet, die mit dem Hydraulikanschluss (149) fluidisch verbunden ist.

2. Werkzeug (14) nach Anspruch 1,
**dadurch gekennzeichnet, dass**
- die Branchen (141') in dem Werkzeuggehäuse (144) schwenkbar auf einer Führungsachse (142) gelagert sind, und/oder
- die Ausnehmung (144) in der Schwenkebene der Branchen (141') liegt.

3. Werkzeug (14) nach Anspruch 1 oder 2,
**dadurch gekennzeichnet, dass**
der Hydraulikanschluss in einen Ballon (152) mündet, der in der Kavität (15) angeordnet, bevorzugt mit den Branchen (141') verbunden, besonders bevorzugt mit den Branchen (141') zumindest punktuell verklebt ist.

4. Werkzeug (14) nach zumindest einem der Ansprüche 1 bis 3,
**dadurch gekennzeichnet, dass**
ein elektrisches Stellglied, bevorzugt eine Pumpe, zum Einstellen des Drucks und der Füllung der Kavität (15) fluidisch mit der Kavität (15), bevorzugt über den Hydraulikanschluss (149), verbunden ist,
wobei die Pumpe bevorzugt an dem Werkzeuggehäuse (144) angeordnet ist und besonders bevorzugt mit einem Ausgleichsbehälter, der an dem Werkzeuggehäuse (144) angeordnet ist, fluidisch verbunden ist.

5. Werkzeug (14) nach zumindest einem der Ansprüche 1 bis 4,
**dadurch gekennzeichnet, dass**
das chirurgische Werkzeug (14) eine Rückstellvorrichtung (146) aufweist, mittels der die Branchen (141') in eine Ruhestellung rückstellbar sind, wobei die Rückstellvorrichtung (146) bevorzugt ein Federring oder ein Elastomerring ist, der besonders bevorzugt kraftleitend mit dem Führungsabschnitt (145") der Branchen (141) verbunden ist.

6. Werkzeug (14) nach zumindest einem der Ansprüche 1 bis 5,
**dadurch gekennzeichnet, dass**
die Branchen (141') in dem Wirkabschnitt (141) Schneiden oder Greifflächen aufweisen.

7. Mikrochirurgisches Instrument (1), das eine proximale Handhabe (11) mit einer Betätigungsvorrichtung (12) und ein distales hydraulisch betätigbares chirurgisches Werkzeug (14) aufweist, das durch einen Schaft mit der Handhabe verbunden ist,
**dadurch gekennzeichnet, dass**
das chirurgische Werkzeug ein chirurgisches Werkzeug (14) nach zumindest einem der Ansprüche 1 bis 6 ist und der Druck und die Füllung der Kavität (15) des chirurgischen Werkzeugs (14) mittels der Betätigungsvorrichtung (12) einstellbar sind.

8. Instrument (1) nach Anspruch 7,
**dadurch gekennzeichnet, dass**
- das chirurgische Werkzeug (14) dauerhaft oder auswechselbar mit dem Schaft (13) verbunden ist, und/oder
- die Handhabe (11) bevorzugt eine Rückstellvorrichtung zur Rückstellung der Betätigungsvorrichtung (12) in eine Ruhestellung aufweist.

9. Instrument (1) nach Anspruch 7 oder 8,
**dadurch gekennzeichnet, dass**
an der Handhabe (11) eine Druckerzeugungsvorrichtung angeordnet ist, die mittels der Betätigungsvorrichtung (12) betätigbar ist und die mittels einer Fluidführungsvorrichtung (16), die bevorzugt ein Rohr oder Schlauch ist, fluidisch mit der Kavität (15) des chirurgischen Werkzeugs (14), bevorzugt mit dem Hydraulikanschluss (149), verbunden ist, wobei die Druckerzeugungsvorrichtung bevorzugt ein fluidgefüllter Ballon ist, der mittels der Betätigungsvorrichtung (12) komprimierbar ist.

10. Instrument nach Anspruch 7 oder 8,
**dadurch gekennzeichnet, dass**
- an der Handhabe (11) zumindest ein elektrisches Messglied angeordnet ist, mit dem zumindest eine Betätigungskraft der Betätigungsvorrichtung (12), bevorzugt eine Betätigungskraft und ein Betätigungsweg, messbar ist/sind und
- das elektrische Messglied operativ mit dem elektrischen Stellglied des chirurgischen Werkzeugs (14) verbunden ist,

11. Instrument nach Anspruch 7 oder 8,
**dadurch gekennzeichnet, dass**
in dem Schaft (13) eine Schubstange längsaxial verschiebbar gelagert ist, die mechanisch mit der Betätigungsvorrichtung (12) gekoppelt ist und sich bis zu dem chirurgischen Werkzeug (14) erstreckt, wobei die Schubstange dazu ausgebildet ist, bei einer Betätigung der Betätigungsvorrichtung (12) entlang eines Längenabschnitts in die Kavität einzutauchen.

12. Instrument nach zumindest einem der Ansprüche 7 bis 11,
**dadurch gekennzeichnet, dass**
der Schaft (13)
- zumindest entlang eines Längenabschnitts biegsam und/oder starr ist und/oder
- längenverstellbar ist, bevorzugt teleskopierbar und/oder
- zumindest ein Gelenk aufweist, bevorzugt ein Gelenk mit zwei Rotationsfreiheitsgraden.

## Claims

1. A surgical tool (14),
comprising a proximal coupling portion (145) from which two hydraulically actuatable, pivotably connected members (141') extend, each having an active portion (141), at least at their distal ends,
wherein
the members (141') in the coupling portion (145) form a boundary for at least a part of a fluid-tight, fluid-fillable cavity (15), such that a pivot position of the members (141') and a volume of the fluid are operatively coupled together in the cavity (15) **characterized in that**
the surgical tool (14) in the coupling portion (145) comprises a tool housing (144) with at least one recess (144') in which the pivotably connected members (141') are led by a guide portion (145"), such that at the tool housing (144) a hydraulic connection (149) is disposed and such that the tool housing (144) forms the cavity (15) with the members (141'), said cavity (15) being fluidically connected with the hydraulic connection (149).

2. The tool (14) according to claim 1,
**characterized in that**
- the members (141') in the tool housing (144) are pivotably disposed on a guide axis (142), and/or
- the recess (144') is situated in the pivot plane of the members (141').

3. The tool (14) according to claim 1 or 2,
**characterized in that**
the hydraulic connection leads into a balloon (152), which is disposed in the cavity (15) and is connected to the members (141'), and is at least glued to the members at selective points.

4. The tool (14) according to at least one of claims 1 to 3,
**characterized in that**
an electrical control element, preferably a pump, to adjust the pressure and the filling of the cavity (15) is fluidically connected with the cavity (15), preferably by the hydraulic connection (149), such that the pump is preferably disposed on the tool housing (144) and is particularly preferably fluidically connected with an expansion reservoir that is disposed on the tool housing (144).

5. The tool (14) according to at least one of claims 1 to 4,
**characterized in that**
the surgical tool (14) comprises a return device (146), by means of which the members (141') can be restored to a resting position, wherein preferably the return device (146) is a spring lock ring or an elastomer ring, which is particularly prefered conductively connected with the guide portion (145") of the members (141').

6. The tool (14) according to at least one of claims 1 to 5,
**characterized in that**
the members (141') in the active portion (141) comprise blades or gripping surfaces.

7. A micro-surgical instrument (1), comprising a proximal handle (11) with an actuation device (12) and comprising a distal hydraulically actuatable surgical tool (14), which is connected by a shaft with the handle,
**characterized in that**
the surgical tool is a surgical tool (14) according to at least one of claims 1 to 6 and the pressure and filling of the cavity (15) of the surgical tool (14) are adjustable by means of the actuation device (12).

8. The instrument (1) according to claim 7,
**characterized in that**
- the surgical tool (14) is connected permanently or intermittently with the shaft (13), and/or
- the handle preferably comprises a return device to restore the actuation device (12) to a resting position.

9. The instrument (1) according to claim 7 or 8,
**characterized in that**
on the handle (11) a pressure-generating device is disposed, which can be actuated by means of the actuation device (12) and which, by means of a fluid feeder device (16) that preferably is a tube or hose, is fluidically connected with the cavity (15) of the surgical tool (14), preferably with the hydraulic connection (149), wherein the pressure-generating device preferably is a fluid-filled balloon, which can be compressed by means of the actuation device (12).

10. The instrument according to claim 7 or 8,
**characterized in that**
- on the handle (11) at least one electrical measurement element is disposed with which at least one actuation force of the actuation device (12), preferably an actuation force and an actuation pathway, can be measured, and
- the electrical measurement element is operatively connected with the electrical adjustment element of the surgical tool (14).

11. The instrument according to claim 7 or 8,
**characterized in that**
in the shaft (13) a push rod is disposed slidably along the longitudinal axis and is mechanically coupled with the actuation device (12) and extends as far as the surgical tool (14), wherein the push rod is configured to dip into the cavity along a longitudinal portion upon actuation of the actuation device (12).

12. The instrument according to at least one of claims 7 to 11,
**characterized in that**
the shaft (13)
- is pliable and/or rigid at least along a longitudinal portion and/or
- is of adjustable length, preferably telescopable, and/or
- comprises at least one joint, preferably a joint with two rotational degrees of freedom.

## Revendications

1. Outil chirurgical (14)
présentant une section de couplage (145) proximale, à partir de laquelle s'étendent deux branches (141') reliées par pivotement, hydrauliquement actionnables, possédant, au moins à chacune de leurs extrémités distales, une section de fonctionnement (141),
les branches (141') formant, dans la section de couplage (145), une limitation pour au moins une partie d'une cavité (15) étanche au fluide, pouvant être remplie de fluide, une position de pivotement des branches (141') et un volume du fluide dans la cavité (15) étant opérationnellement couplés,
**caractérisé en ce que**
l'outil chirurgical (14) présente, dans la section de couplage (145), un boîtier d'outil (144) avec au moins un évidement (144') dans lequel les branches (141') reliées par pivotement sont guidées avec une section de guidage (145"), un raccord hydraulique (149) étant disposé sur le boîtier d'outil (144) et le boîtier d'outil (144) avec les branches (141') formant la cavité (15) qui est reliée fluidiquement au raccord hydraulique (149).

2. Outil (14) conformément à la revendication n°1,
**caractérisé en ce que**
- les branches (141') sont montées en pivotement sur un axe de guidage (142) dans le boîtier d'outil (144) et/ou
- l'évidement (144) se trouve dans le plan de pivotement des branches (141').

3. Outil (14) conformément à la revendication n°1 ou n°2,
**caractérisé en ce que**
le raccord hydraulique débouche dans un ballon (152) qui est disposé dans la cavité (15), de préférence relié aux branches (141'), en particulier de préférence collé au moins ponctuellement avec les branches (141').

4. Outil (14) conformément au moins à l'une des revendications n°1 à n°3,
**caractérisé en ce que**
un actionneur électrique, de préférence une pompe, pour le réglage de la pression et le remplissage de la cavité (15), est relié fluidiquement à la cavité (15), de préférence par le raccord hydraulique (149),
la pompe étant disposée de préférence sur le boîtier d'outil (144) et en particulier de préférence relié fluidiquement à un réservoir de compensation qui est disposé sur le boîtier d'outil (144).

5. Outil (14) conformément au moins à l'une des revendications n°1 à n°4,
**caractérisé en ce que**
l'outil chirurgical (14) présente un dispositif de réinitialisation (146) permettant de réinitialiser les branches (141') dans une position de repos, le dispositif de réinitialisation (146) étant de préférence une rondelle-ressort ou une bague élastomère qui est reliée en particulier de préférence avec conduction de force à la section de guidage (145") des branches (141).

6. Outil (14) conformément au moins à l'une des revendications n°1 à n°5,
**caractérisé en ce que**
les branches (141') présentent, dans la section de fonctionnement (141), des bords tranchants ou des surfaces de préhension.

7. Instrument microchirurgical (1) présentant une poignée (11) proximale avec un dispositif d'actionnement (12) et un outil chirurgical (14) distal, actionnable hydrauliquement, lequel est relié à la poignée par une tige,
**caractérisé en ce que**
l'outil chirurgical est un outil chirurgical (14) conformément au moins à l'une des revendications n°1 à n°6 et que la pression et le remplissage de la cavité (15) de l'outil chirurgical (14) sont réglables au moyen du dispositif d'actionnement (12).

8. Instrument (1) conformément à la revendication n°7,
**caractérisé en ce que**
- l'outil chirurgical (14) est relié à la tige (13) durablement ou de manière interchangeable et/ou
- la poignée (11) présente de préférence un dispositif de réinitialisation pour la réinitialisation du dispositif d'actionnement (12) dans une position de repos.

9. Instrument (1) conformément à la revendication n°7 ou n°8,
**caractérisé en ce que**
un dispositif de génération de pression qui est actionnable au moyen du dispositif d'actionnement (12) et qui est relié fluidiquement à la cavité (15) de l'outil chirurgical (14), de préférence au raccord hydraulique (149), au moyen d'un dispositif de guidage du fluide (16) qui est de préférence un tube ou tuyau, est disposé sur la poignée (11), le dispositif de génération de pression étant de préférence un ballon rempli de fluide qui est comprimable au moyen du dispositif d'actionnement (12).

10. Instrument conformément à la revendication n°7 ou n°8,
**caractérisé en ce que**
- au moins un organe de mesure électrique, permettant de mesurer au moins une force d'actionnement du dispositif d'actionnement (12), de préférence une force d'actionnement et une course d'actionnement, est disposé sur la poignée (11) et
- l'organe de mesure électrique est opérationnellement relié à l'actionneur électrique de l'outil chirurgical (14).

11. Instrument conformément à la revendication n°7 ou n°8,
**caractérisé en ce que**
une bielle de poussée qui est mécaniquement couplée avec le dispositif d'actionnement (12) et qui s'étend jusqu'à l'outil chirurgical (14), est logée de manière coulissante en sens axial longitudinal dans la tige (13), la bielle de poussée étant conçue pour plonger dans la cavité lors d'un actionnement du dispositif d'actionnement (12) le long d'une section de longueur.

12. Instrument conformément au moins à l'une des revendications n°7 à n°11,
**caractérisé en ce que**
la tige (13)
- est flexible et/ou rigide au moins le long d'une section de longueur et/ou
- est réglable en longueur, de préférence télescopable et/ou
- présente au moins une articulation, de préférence une articulation avec deux degrés de liberté de rotation.
